# EUROPÄISCHE PATENTSCHRIFT

(11) **EP 0 863 774 B1**
(45) Veröffentlichungstag und Bekanntmachung des Hinweises auf die Patenterteilung: **10.11.2004**
(21) Anmeldenummer: 96922024.3
(22) Anmeldetag: 24.06.1996
(51) Int. Cl.: A61L 15/44, A61M 35/00, A61K 9/70

(54) **TRANSDERMALES THERAPEUTISCHES SYSTEM ZUR ABGABE VON WIRKSTOFFEN ÜBER DIE HAUT AN DEN MENSCHLICHEN KÖRPER**
TRANSDERMAL THERAPEUTIC SYSTEM FOR ADMINISTERING ACTIVE AGENTS TO THE HUMAN BODY VIA THE SKIN
SYSTEME THERAPEUTIQUE TRANSDERMIQUE POUR ADMINISTRER DES CONSTITUANTS ACTIFS AU CORPS HUMAIN PAR VOIE CUTANEE

(30) Priorität: 29.07.1995 DE 19527925
(43) Veröffentlichungstag der Anmeldung: 16.09.1998
(73) Patentinhaber: LTS Lohmann Therapie-Systeme AG, 56626 Andernach (DE)
(72) Erfinder: HORSTMANN, Michael, D-56564 Neuwied (DE); LEONHARD, Johannes, D-56170 Bendorf (DE); MÜLLER, Walter, D-56564 Neuwied (DE)
(74) Vertreter: Flaccus, Rolf-Dieter
(86) Internationale Anmeldenummer: PCT/EP1996/002735
(87) Internationale Veröffentlichungsnummer: WO 1997/004818

(56) Entgegenhaltungen:
- WO-A-96/40259
- FR-A- 2 397 190
- GB-A- 1 414 812
- US-A- 3 797 494
- US-A- 5 071 704
- US-A- 5 186 939
- J. CONTROLLED RELEASE, Bd. 29, Nr. 1/2, Februar 1994, AMSTERDAM, Seiten 177-185, XP002023290 SUGIBAYASHI K. ET AL: "Polymers for transdermal drug delivery systems"

## Beschreibung

Die Erfindung betrifft ein transdemales therapeutisches System zur Abgabe von Wirkstoffen über die Haut an den menschlichen Körper, insbesondere ein transdermales therapeutischs System mit geschichtetem Aufbau aus einer Rückschicht (1) und mindestens einer Wirkstoff im übersättigten Zustand enthaltenden Matrixschicht (2), zur Lagerung aufgelegt auf einer mit einem Trennmittel (3) vorbeschichteten ablösbaren Schutzschicht (4), dadurch gekennzeichnet, daß das Trennmittel (3) einen geringeren Diffusionskoeffizienten für den verwendeten Wirkstoff aufweist als die in der Matrixschicht (2) oder in den Matrixschichten verwendeten Grundmaterialien, wobei die Grundmaterialien der Matrixschicht (2) oder der Matrixschichten zum überwiegenden Teil aus wasserunlöslichen, hautverträglichen Polymeren und für den Hautkontakt geeigneten Harzbestandteilen bestehen und die Polymere Copolymere aus Dienen, Styrol, Polyisobutylene oder Naturkautschuk sind.

Transdermale therapeutische Systeme (TTS) sind in der arzneilichen Therapie einer Reihe von Erkrankungen bereits im Markt eingeführt.

Ein Nachteil der dem Stand der Technik entsprechenden Systeme ist die nicht ausreichende Permeationsfähigkeit vieler Wirkstoffe durch die Haut, die auch durch zahlreiche galenische Maßnahmen des TTS-Designs (Einsatz mehrschichtiger Systeme, Verwendung von Steuermembranen, Variation der Wirkstoffkonzentration, Modifikation der Grundpolymeren etc.) nicht über eine gewisse Grenze, den sog. "Sättigungsfluß", hinaus steigerbar ist. Die Feststellung, daß der transdermale Fluß eines Wirkstoffes aus der festen, fein verteilten Phase heraus auch bei Einsatz stärker lösender Vehikel prinzipiell nicht weiter steigerbar ist, findet sich bereits in den auch heute noch wegweisenden Arbeiten von Higuchi (z.B. T. Higuchi: Physical Chemical Analysis of percutaneous absorptions process from creams and ointments. J. Soc. Cosmetic Chem, 11, S. 85-97 (1960).

Allerdings gibt es für viele Wirkstoffe die Möglichkeit, dem TTS bei der Herstellung Penetrationsverstärker, sogenannten "Enhancer" zuzusetzen. Es handelt sich in der Regel um flüssige bzw. flüchtige Zusatzstoffe, die die Resorptionseigenschaften der menschlichen Haut verbessern und damit eine genügend intensive Aufnahme des Wirkstoffs aus einer relativ kleinen TTS-Fläche heraus ermöglichen. Jedoch ergeben leicht flüchtige Enhancer wie das zum Beispiel für den Wirkstoff Estradiol verwendete Ethanol Probleme infolge starker Erweichung der Klebeschichten von TTS und machen dadurch weitere, raumfordernde Kompartimente im System notwendig, die das TTS unakzeptabel dick oder großflächig werden lassen. Schließlich ergibt jeder weitere nichtpolymere Zusatzstoff auch die Gefahr von Unverträglichkeiten auf der Haut, unter Umständen auch von Sensibilisierungen. Unter Zusatz bestimmter, weniger flüchtiger, meist aber auch weniger aktiver Enhancer (z.B. Glycerinester, cyclische Amide, Eucalyptol) ist zwar die Herstellung von Matrixsystemen möglich, die den Wirkstoff und die resorptionsverstärkende Komponente in einer oder mehreren monolithischen Schichten enthalten.
Nach dem Stand der Technik ist deshalb solchen TTS eine befriedigende Therapie dann nicht möglich, wenn die Enhancer schlecht hautverträglich sind oder die Systeme wegen des noch immer zu geringen Flusses durch die Haut unakzeptabel große Flächen benötigen.

Eine andere Möglichkeit, den Wirkstofffluß durch die Haut zu steigern, besteht darin, mehr Wirkstoff im TTS molekulardispers aufzulösen als der Sättigungslöslichkeit entspricht. Mit einer solchen Übersättigung dieser Systeme steigt in gleichem Maße auch die Permeationsgeschwindigkeit durch die Haut an. Da solche Zustände jedoch thermodynamisch instabil sind, sind derartige Arzneiformen nur schwer in lagerstabiler Form bereitzustellen; es findet eine zeitlich nicht vorhersehbare Rekristallisation von wirkstoffteilchen statt, so daß die Flußrate durch die Haut nach und nach auf Sättigungsflußniveau abfällt und damit je nach Ausgangskonzentration ein großer Teil der initial vorhandenen therapeutischen Aktivität verlorengeht.

In vielen Fällen solcher Rekristallisation konnte mikroskopisch beobachtet werden, daß Kristallisationen ihren Ursprung nicht in den Komponenten des TTS hatten, sondern von der Trennmittelschicht ausgingen.

Ausgehend vom vorgenannten Stand der Technik ist es Aufgabe der Erfindung, ein transdermales therapeutisches System anzugeben, welches einen geschichteten Aufbau aus einer Rückschicht und mindestens einer wirkstoffhaltigen Matrixschicht aufweist und bei Lagerung in Kontakt mit einer mit einem Trennmittel vorbeschichteten ablösbaren Schutzschicht keine oder nur unerhebliche vorzeitige Ausfällung des Wirkstoffes erfährt.

Diese Aufgabe wird erfindungsgemäß dadurch gelöst, daß das Trennmittel einen geringeren Diffusionskoeffizienten für den verwendeten Wirkstoff als die in der Matrixschicht oder in den Matrixschichten verwendeten Grundmaterialien aufweist. Insbesondere wird dies mit fluorhaltigen Polymeren als Bestandteilen der Trennschicht erreicht.

Ein fluorhaltiges Polymer wird in der Offenlegungsschrift FR 2 397 190, in der die Herstellung eines transdermalen therapeutischen Systems beschrieben wird, welches aus einer Rückschicht, einer Matrixschicht, einer Steuermembran und einer Schutzschicht besteht, als Material für die Steuermembran offenbart. Zusätzlich offenbart FR 2 397 190 eine Beschichtung auf Siliconbasis als Trennmittel für die Schutzschicht.

US Patent Nr. 5,186,939 und die nicht vorveröffentlichte WO 96/40259 offenbaren transdermale therapeutische Systeme, die einen Wirkstoff in übersättigtem Zustand in einer Matrix, die zum überwiegenden Teil aus Polysiloxan bzw. Polydimethylsiloxan besteht, sowie eine Schutzschicht aus einer mit einem fluorhaltigen Polymer beschichteten Polyesterfolie (3M - 1022) umfassen.

Durch Reihenversuche mit verschiedenen Paarungen zwischen ein- und mehrschichtigen Matrices sowie mit durch unterschiedliche Trennmittel beschichteten Schutzschichten wurde überraschend erkannt, daß Rekristallisationen sich regelmäßig verlangsamen oder sogar komplett ausbleiben, wenn Trennmittel mit einem geringen Diffusionskoeffizienten für den Wirkstoff gewählt werden.

So erweisen sich im erfindungsgemäßen Sinne fluorhaltige Polymere als besonders geeignet, die neben den üblicheren siliconbasierten Trennmitteln in ausreichender Qualität auf dem Markt verfügbar sind. So sind solche Trennmittel bereits im vorgefertigten Verbund mit der in der Regel dikkeren Schutzfolie in großer Auswahl verfügbar. Die Verwendung von mit Trennmitteln beschichteten Folien ist gegenüber dem Einsatz von Vollschichten des Trennmittels vorzuziehen, da eine Kostenersparnis des oftmals teueren Trennmittels eintritt oder aber sich Festigkeits-Vorteile ergeben. Es ist jedoch im Sinne dieser Erfindung durchaus auch möglich, die Schutzschicht einschließlich Trennschicht aus einem einheitlichen Material bereitzustellen.
Die besonders bevorzugten Fluorpolymere können im einzelnen aus Polytetrafluorethylen, Perfluorethylenpropylen-Copolymeren, Perfluoralkoxy-Copolymeren, Polychlortrifluorethylen, Ethylen-Tetrafluorethylen-Copolymeren, Ethylen-Chlortrifluorethylen-Copolymeren, Polyvinylidenfluorid oder auch Polyvinylfluorid bestehen, wobei diese Aufzählung nicht erschöpfend ist und nur eine Auswahl der Möglichkeiten illustrieren soll.

Weiterhin können die Trennmittel aber auch aus anderen im Vergleich zum Matrix-Grundmaterial gering diffusiblen Stoffen bestehen, wie zum Beispiel Polyethylen, Polypropylen, Polyvinylchlorid oder Polyvinylidenchlorid, sofern sie ausreichende Trenneigenschaften besitzen.

Als Grundmaterial erfindungsgemäßer TTS können neben den weitverbreiteten Acrylsäureestercopolymeren auch andere Polymere, wie Polyisobutylen, Polyvinylacetat und Copolymere, Synthesekautschuk, Silicone verwendet werden. Sofern zum Beispiel zur Erzielung einer hinreichenden Klebkraft erforderlich, können Mischungen solcher und anderer Polymere mit Zuschlagstoffen, z.B. Harzen, hautkompatiblen Ölkomponenten, Füllstoffen etc. verwendet werden, wobei die Grundmaterialien der Matrixschicht oder der Matrixschichten zum überwiegenden Teil aus wasserunlöslichen, hautverträglichen Polymeren und für Hautkontakt geeigneten Harzbestandteilen bestehen.

Am deutlichsten tritt der erfindungsgemäße Effekt jedoch auf, wenn Copolymere aus Dienen und Styrol, Polyisobutylene oder Naturkautschuk verwendet und Derivate natürlicher Harze oder Kohlenwasserstoffharze als Klebrigmacher zugesetzt werden.

Der Vorteil der Erfindung kann insbesondere bei Wirkstoffen beobachtet werden, die bei der vorgesehenen Lagertemperatur fest sind. Unter diesen sind beispielsweise besonders hervorzuheben:
zentral wirksame Substanzen wie zum Beispiel Amantadin, Benzatropin, Biperiden, Bornaprin, Trihexyphenidyl, Tranylcypromin, Physostigmin, Selegilin, Doxepin, Maprotilin, Imipramin, Perphenazin, Haloperidol, Benperidol, Sulpirid, Pimozid, Methylphenidat, Amfetamimil, Amphetamin, Cocain, Oxazepam, Alprazolam, Diazepam, Lorazepam, Buspiron, Xanomelin, Piracetam, Ephedrin, Norpseudoephedrin, Fenproporex, Fenfluramin,
opioide Analgetika wie Morphin, Heroin, Tilidin, Alfentanil, Methadon, Sufentanil, Fentanyl,
peripher wirkende Analgetika wie Ketorolac, Ketoprofen, Indomethacin, Acetylsalicylsäure, Diclofenac, Tenoxicam, Antikoagulantien wie Warfarin, Phenprocoumon, Acetylsalicylsäure, Acenocoumarol,
Antihistaminika wie Pheniramin, Chlorpheniramin, Terfenadin, Trimetinden, Prednisolon, Bamipin, Clemastin, Steroidhormone, zum Beispiel für postmenopausalen, anabolen, kontrazeptiven oder antiinflammatorischen Einsatz wie Medroxyprogesteron, Levonorgestrel, Testosteron, Metenolon, Nandrolon, Androsteron, Cyproteronacetat, Medroxyprogesteronacetat, Lynestrenol, Norethisteron, Epimestrol, Estriol, Estron, Estradiolvalerat, Estradiolpropionat, Norethiseronacetat, Norgestrel, Gestoden, Mestranol, Estradiol, Ethinylestradiol,
Prostaglandine, wie Gemeprost, Dinoproston, Sulproston, osteoprotektive Stoffe wie Vitamin D3, Raloxifen, Etidronsäure,
blutdrucksenkende Stoffe wie Enalapril, Captopril, Moxonidin, Clonidin, Timolol, Propanolol, Bupranolol, Bopindolol, Metoprolol, Pindolol, Mepindolol,
sympathotonische Substanzen wie Etilefrin, Ephedrin, Midodrin,
Antiallergika wie Phenuramin, Brompheniramin, Ketotifen, Terfenadin, Dimetinden, Cyproheptadin,
Lokalanästhetika wie Bupivacain, Mepivacain, Lidocain, Butanilicain,
Asthmamittel wie Salbutamol, Clenbuterol, Tulobuterol, Atropin, Scopolamin, Fenoterol
und viele andere Wirkstoffe, die hier im einzelnen nicht weiter aufgeführt werden.

Die Diffusibilität des Trennmittels soll erfindungsgemäß geringer sein als diejenige des Grundmaterials der Matrixschichten. In der Literatur sind eine Reihe von Methoden zur Bestimmung des Diffusionskoeffizienten beschrieben, welche sich aus den Fickschen Gesetzen ableiten. So ist es zum Beispiel möglich, mit Wirkstoff vorbeladene Schichten des Trennmittels oder aber eines Matrixgrundmaterials bei festgelegter Temperatur in einer Diffusionszelle zu untersuchen, in welcher die freigesetzte Substanzmenge in Abhängigkeit von der Zeit bestimmt werden kann. Das Verfahren der Bestimmung des Diffusionskoeffizienten ist zum Beispiel nach Kokubo et al., Proceed. Intern. Symp. Control, Rel. Bioact. Mater. 17 (1990), S. 271-272 durchführbar. Der sich ergebende Diffusionskoeffizient des Trennmittels soll bei erfindungsgemäßen TTS niedriger sein als der Diffusionskoeffizient der Matrix.

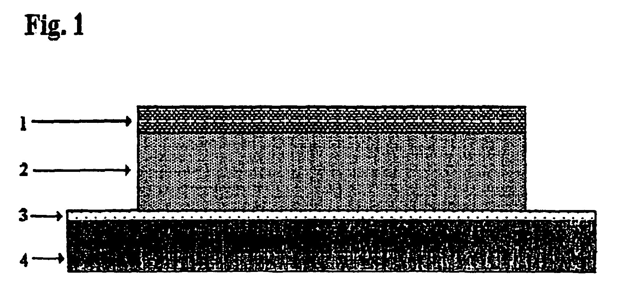

In Fig. 1 ist beispielhaft ein erfindungsgemäßes System, bestehend aus Rückschicht (1), Matrix (2), Trennmittel (Trennschicht) (3) und Schutzschicht (4) dargestellt. Die Matrix (2) kann auch mehrschichtig ausgebildet sein.

### Beispiel 1:

Herstellung eines erfindungsgemäßen Systems

| | |
|---|---|
| 2,0 g | 17-β-Estradiol-semihydrat, mikronisiert |
| 60,0 g | Cariflex **®** TR 1107 (Styrol-Isopren-Styrol-Blockcopolymer) |
| 120,0 g | Staybelite Ester 5E (thermoplastisches Esterharz aus Kolophoniumderivaten ) |
| 20 g | dickflüssiges Paraffin |

werden bei Raumtempertur in einem zylindrischen Glasgefäß bis zum Erreichen einer gleichmäßigen Suspension gerührt und anschließend mit einer Spaltbreite von 500 Mikrometern auf 100 Mikrometer dicke, mit 2 g/m² Silikonkautschuk vorbeschichtete Polyesterfolie beschichtet. Der Ausstrich wird je zehn Minuten lang bei 25°C, bei 50°C, bei 80°C und bei 95°C getrocknet. Sofort wird 15 Mikrometer dicke Polyesterfolie luftblasenfrei unter Walzendruck auf die getrocknete Schicht aufgelegt (zukaschiert).
Durch Stanzung mit Henkellocheisen werden Transdermale Systeme von 10 cm² erhalten, die in Verbundpackstoff aus Papier/Aluminiumfolie/Heißsiegelschicht unter Zugabe einer Trockentablette, enthaltend 0,3 Calciumsulfat (welches zuvor bei 180°C vorgetrocknet wurde) verpackt werden.
Anschließend wird 15 Mikrometer dicke Polyesterfolie luftblasenfrei unter Walzendruck auf die noch warme Schicht aufgelegt (zukaschiert).
Durch Stanzung mit Henkellocheisen werden Transdermale Systeme von 20 cm² erhalten.

### Beispiel 2:

Vergleichsbeispiel zu 1

| | |
|---|---|
| 2,0 g | 17-β-Estradiol-semihydrat, mikronisiert |
| 60,0 g | Cariflex ^{R} TR 1107 (Styrol-Isopren-Styrol-Blockcopolymer |
| 120,0 g | Staybelite Ester 5E (thermoplastisches Esterharz aus Kolophoniumderivaten) |
| 20 g | dickflüssiges Paraffin |

werden bei Raumtemperatur in einem zylindrischen Glasgefäß bis zum Erreichen einer gleichmäßigen Suspension gerührt und anschließend mit einer Spaltbreite von 500 Mikrometern auf 100 Mikrometer dicke, mit Fluorpolymer vorbeschichtete Polyesterfolie (Scotchpak ® 1022) beschichtet. Der Ausstrich wird je zehn Minuten lang bei 25°C, bei 50°C, bei 80°C und bei 95°C getrocknet. Sofort wird 15 Mikrometer dicke Polyesterfolie luftblasenfrei unter Walzendruck auf die getrocknete Schicht aufgelegt (zukaschiert).
Durch Stanzung mit Henkellocheisen werden Transdermale Systeme von 10 cm² erhalten, die in Verbundpackstoff aus Papier/Aluminiumfolie/Heißsiegelschicht unter Zugabe einer Trockentablette, enthaltend 0,3 g Calciumsulfat (welchs zuvor bei 180°C vorgestrocknet wurde, verpackt werden. Anschließend wird 15 Mikrometer dicke Polyesterfolie luftblasenfrei unter Walzendruck auf die noch warme Schicht aufgelegt (zukaschiert).
Durch Stanzung mit Henkellocheisen werden Transdermale Systeme von 20 cm² erhalten.

## Patentansprüche

1. Transdermales therapeutisches System mit geschichtetem Aufbau aus einer Rückschicht (1) und mindestens einer Wirkstoff im übersättigten Zustand enthaltenden Matrixschicht (2), zur Lagerung aufgelegt auf einer mit einem Trennmittel (3) vorbeschichteten ablösbaren Schutzschicht (4), **dadurch gekennzeichnet, daß** das Trennmittel (3) einen geringeren Diffusionskoeffizienten für den verwendeten Wirkstoff aufweist als die in der Matrixschicht (2) oder in den Matrixschichten verwendeten Grundmaterialien, wobei die Grundmaterialien der Matrixschicht (2) oder der Matrixschichten zum überwiegenden Teil aus wasserunlöslichen, hautverträglichen Polymeren und für den Hautkontakt geeigneten Harzbestandteilen bestehen und die Polymere Copolymere aus Dienen, Styrol, Polyisobutylene oder Naturkautschuk sind.

2. Transdermales therapeutisches System nach Anspruch 1, **dadurch gekennzeichnet, daß** der Diffusionskoeffizient des Trennmittels (3) für den Wirkstoff höchstens bei einem Zehntel des Diffusionskoeffizienten der Grundmaterialien der Matrixschicht (2) oder der Matrixschichten liegt.

3. Transdermales therapeutisches System nach Anspruch 1 oder 2, **dadurch gekennzeichnet, daß** das Trennmittel ein fluorhaltiges Polymer ist.

4. Transdermales therapeutisches System nach Anspruch 1, **dadurch gekennzeichnet, daß** das Trennmittel zwischen 0,1 bis 5 µm als zusammenhängende Schicht (3) auf der Schutzschicht aufgebracht ist.

5. Transdermales therapeutisches System nach Anspruch 1, **gekennzeichnet dadurch, daß** die genannten Harzbestandteile Derivate natürlicher Harze oder Kohlenwasserstoffderivate sind.

6. Transdermales therapeutisches System nach einem oder mehreren der vorhergehenden Ansprüche, **gekennzeichnet dadurch, daß** es sich bei mindestens einem Wirkstoff um ein Steroidhormon handelt.

## Claims

1. Transdermal therapeutic system having a layered structure comprising a backing layer (1) and at least one matrix layer (2) containing active substance in an oversaturated state, for storage purposes being placed on a removable protective layer (4) precoated with an abherent (3), **characterized in that** said abherent (3) has a lower diffusion coefficient for the active substance used than the base materials used in the matrix layer (2) or matrix layers, with said base materials of the matrix layer (2) or matrix layers predominantly comprising water-insoluble, skin-tolerated polymers and resin components suitable for skin contact, and the polymers consisting of dienes, styrene, polyisobutylene or natural rubber.

2. Transdermal therapeutic system according to claim 1, **characterized in that** the diffusion coefficient of the abherent (3) for the active substance amounts to at most one tenth of the diffusion coefficient of the base materials of the matrix layer (2) or of the matrix layers.

3. Transdermal therapeutic system according to claim 1 or 2, **characterized in that** the abherent is a fluorine-containing polymer.

4. Transdermal therapeutic system according to claim 1, **characterized in that** the abherent is applied to the protective layer in a thickness of between 0.1 to 5 µm, as a continuous layer (3).

5. Transdermal therapeutic system according to claim 1, **characterized in that** the resin components mentioned are derivatives of natural resins or hydrocarbon derivatives.

6. Transdermal therapeutic system according to one or more of the preceding claims, **characterized in that** at least one active substance is a steroid hormone.

## Revendications

1. Système thérapeutique transdermique avec une structure stratifiée constituée d'une couche de recouvrement (1) et au moins une couche de matrice (2) contenant au moins une substance active à l'état sursaturé, appliqué en vue d'un entreposage sur une couche protectrice (4) détachable revêtue au préalable d'un agent de séparation (3), **caractérisé en ce que** l'agent de séparation (3) présente un coefficient de diffusion pour la substance active employée, plus faible que celui des matières de base employées dans la couche de matrice (2) ou dans les couches de matrice, les matières de base de la couche de matrice (2) ou des couches de matrice étant constituées pour une part majoritaire de polymères insolubles dans l'eau, compatibles avec la peau et de composants à base de résine convenant au contact avec la peau, et les polymères étant des copolymères de diènes, styrène, polyisobutylène ou caoutchouc naturel.

2. Système thérapeutique transdermique selon la revendication 1, **caractérisé en ce que** le coefficient de diffusion de l'agent de séparation (3) pour la substance active, est au plus d'un dixième du coefficient de diffusion des matières de base de la couche de matrice (2) ou des couches de matrice.

3. Système thérapeutique transdermique selon la revendication 1 ou 2, **caractérisé en ce que** l'agent de séparation est un polymère fluoré.

4. Système thérapeutique transdermique selon la revendication 1, **caractérisé en ce que** l'agent de séparation compris entre 0,1 et 5 µm est appliqué sur la couche protectrice en tant que couche (3) contiguë.

5. Système thérapeutique transdermique selon la revendication 1, **caractérisé en ce que** les composants à base de résine cités sont des dérivés de résines naturelles ou des dérivés hydrocarbonés.

6. Système thérapeutique transdermique selon l'une ou plusieurs des revendications précédentes, **caractérisé en ce qu'**il s'agit dans le cas d'au moins une substance active d'une hormone stéroïde.
